# EUROPEAN PATENT APPLICATION

(11) **EP 4 722 349 A1**
(43) Date of publication of application: **08.04.2026**
(21) Application number: 24815636.6
(22) Date of filing: 31.05.2024
(51) Int. Cl.: C12N 5/0783, A61K 35/17, A61K 35/545, A61K 47/68, A61P 35/00, A61P 37/04, C12N 5/10, C12N 15/12

(54) **PLURIPOTENT STEM CELL-DERIVED CD4-POSITIVE T CELLS, METHOD FOR PRODUCTION THEREOF, AND USE THEREOF**

(30) Priority: 31.05.2023 JP 2023089785
(71) Applicant: Juntendo Educational Foundation, Tokyo 113-8421 (JP)
(72) Inventor: ANDO, Miki, Tokyo 113-8421 (JP); ANDO, Jun, Tokyo 113-8421 (JP); ISHII, Midori, Tokyo 113-8421 (JP); FURUKAWA, Yoshiki, Tokyo 113-8421 (JP); NAKAUCHI, Hiromitsu, Tokyo 113-8510 (JP)
(74) Representative: Maiwald GmbH
(86) International application number: PCT/JP2024/020126
(87) International publication number: WO 2024/248157

(57) **Abstract**

The present invention provides a method for producing a CD4 positive T cell, including a step of knocking out one or more genes selected from the group consisting of RUNX3, ZBTB7B, SOCS1, SOCS3, and GIMAPS and/or overexpressing the RUNX1 gene in a human induced pluripotent stem cell (iPSC), a CD4 positive T cell that can be obtained by the method, and a pharmaceutical composition such as immunotherapeutic agent and the like containing the cell.

## Description

### [Technical Field]

The present invention relates to a method for producing CD4 positive T cells, particularly CD4 single-positive (SP) T cells, via pluripotent stem cells, CD4 positive T cells that can be obtained by the method, and uses of the T cells, particularly the use as a cellular immunotherapeutic agent.

### [Background Art]

While adoptive immune cell therapy using T cells has been shown to be effective, it is performed using autologous transplantation in principle, which poses problems such as limited number of cells that can be produced, time-consuming production, and exhaustion of T cells. It has been reported that when antigen-specific T cells are induced, cloned, and then reprogrammed to produce induced pluripotent stem cells (iPSCs), the genomic structure of the rearranged T cell receptor (TCR) gene is inherited (Patent Literature 1, Patent Literature 2). To date, methods have been reported in which iPSCs produced from antigen-specific T cells are induced to differentiate into T cells that exhibit the same antigen specificity as the cells from which they were derived, whereby rejuvenated antigen-specific T cells are produced in large amounts (Patent Literature 1, Patent Literature 2). It has also been reported that large amounts of rejuvenated antigen-specific T cells can be produced from iPSCs produced from peripheral blood-derived T cells with no antigen specificity. However, although these methods can consistently induce CD8-positive (CD8+) T cells, induction of differentiation of iPSCs (CD4T-iPSCs) established from peripheral blood CD4-positive (CD4+) T cell clones fails to stably induce CD4+ T cells, and in reality, the induction requires artificial introduction of the CD4 gene into iPSCs (Patent Literature 3). CD8-positive cytotoxic T cells (CTLs) directly attack cancer, but CD4-positive helper T (Th) cells, particularly Th1 cells, which activate CTL and suppress the proliferation and accumulation of regulatory T cells, which is one of the mechanisms of cancer immune escape, in an interferon (IFN)-γ-dependent manner, are extremely important in T cell immunotherapy.

On the other hand, the present inventors established iPSCs (ATL-iPSCs) from adult T-cell leukemia (ATL) cells and attempted to induce differentiation of the ATL-iPSCs into T cells again, and succeeded for the first time in inducing CD4+CD25+FOXP3+ regulatory T cells (Patent Document 4). However, because this T cell is CD4+CD25+FOXP3+ and regulatory T cell, the use thereof is limited.

### [Citation List]

### [Patent Literature]

[Patent Literature 1]
   WO 2011/096482
[Patent Literature 2]
   JP 6164746 B2
[Patent Literature 3]
   WO 2018/168829
[Patent Literature 4]
   JP 2021-69322 A

### [Summary of Invention]

### [Technical Problem]

The present invention aims to provide a method for efficiently producing non-regulatory CD4+ T cells via pluripotent stem cells. In addition, the present invention aims to provide a novel and effective T cell immunotherapy using the CD4+ T cells.

### [Solution to Problem]

The present inventors compared the gene expression of ATL-iPSC-derived CD4+ T cells described in the aforementioned Patent Literature 3 with that of CD4T-iPSC-derived CD8+ T cells by using single-cell RNA sequencing analysis, and found that CD4T-iPSC-derived CD8+ T cells show differential expression of six genes compared with ATL-iPSC-derived CD4+ T cells. The present inventors then successfully induced differentiation into CD4+ T cells by knocking out one of those genes, RUNX3, in T-iPSCs. Furthermore, they confirmed that forced expression of RUNX3 in ATL-iPSCs results in differentiation into CD8+ T cells, thus demonstrating that the expression level of RUNX3 determines the induction of differentiation into CD4/CD8. They also confirmed that CD4+ T cells induced from T-iPSCs are not regulatory T cells. CD4+ T cells induced from CD8T-iPSCs were CD8-negative but maintained MHC class I-restricted antigen specificity, produced perforin and granzyme B, and exhibited antigen-specific cytotoxic activity. On the other hand, the CD4+ T cells abundantly secreted various cytokines, including Th1 cytokines such as IFNγ and IL-2, and also exhibited a Th1 cell phenotype in expression analyses of transcription factors and cell surface molecules. When co-cultured with cancer antigen-specific CTLs or CAR-T cells, CD4+ T cells induced from T-iPSCs enhanced the cancer antigen-specific cytotoxic activity (tumor growth suppression effect) thereof both in vitro and in vivo.

Based on these findings, the present inventors have conducted further studies and completed the present invention.

That is, the present invention relates to the following [1] to [18].
[1] A method for producing an iPSC-derived CD4+ T cell, comprising a step of knocking out one or more genes selected from the group consisting of RUNX3, ZBTB7B, SOCS1, SOCS3, and GIMAP5 and/or overexpressing the RUNX1 gene in a human iPSC.
[2] The method of [1], wherein the iPSC is derived from an antigen-specific T cell, a peripheral blood-derived T cell, a peripheral blood mononuclear cell, or a fibroblast.
[3] The method of [1] or [2], wherein the gene to be knocked out includes RUNX3.
[4] The method of any one of [1] to [3], wherein the CD4+ T cell is CD4 single-positive.
[5] The method of any one of [2] to [4], wherein the antigen-specific T cell is specific for an antigen peptide presented on a cancer cell or a virus-infected cell, or the peripheral blood-derived T cell or peripheral blood mononuclear cell is derived from a cancer patient or a virus-infected individual.
[6] The method of any one of [2] to [5], wherein the antigen-specific T cell or peripheral blood-derived T cell is a CD8+ T cell.
[7] The method of any one of [2] to [5], wherein the antigen-specific T cell or peripheral blood-derived T cell is a CD4+ T cell.
[8] A human CD4+ T cell obtained by the method of any one of [1] to [7].
[9] A human iPSC-derived T cell having the following properties:
   (a) being CD4 single-positive;
   (b) having a TCR specific to an antigen peptide presented on a cancer cell or a virus-infected cell;
   (c) exhibiting one or more of the following Th1 cell phenotypes:
      (c1) highly secreting IFNγ and IL-2;
      (c2) highly expressing one or more genes selected from the group consisting of TBX21, STAT1, and STAT4;
      (c3) expressing CD107a and CD366;
   (d) independently having cytotoxic activity specific to the aforementioned antigen peptide of (b); and
   (e) enhancing the proliferation and/or cytotoxic activity of cancer antigen- or viral antigen-specific CTL or CAR-T cell.
[10] The human iPSC-derived T cell of [9], in which one or more genes selected from the group consisting of RUNX3, ZBTB7B, SOCS1, SOCS3, and GIMAP5 are knocked out and/or the RUNX1 gene is overexpressed.
[11] A pharmaceutical composition comprising the human iPSC-derived CD4+ T cell of any one of [8] to [10].
[12] The pharmaceutical composition of [11], which is an immunotherapeutic agent.
[13] The pharmaceutical composition of [12], which is for treating cancer or a viral infectious disease.
[14] The pharmaceutical composition of [13], which is combined with cancer antigen- or viral antigen-specific CTL or CAR-T cell.
[15] The pharmaceutical composition of [13], which is combined with an antigen-presenting cell and/or an antigen peptide presented on a cancer cell or a virus-infected cell.
[16] A method for treating cancer or a viral infectious disease in a subject, comprising administering to the subject an effective amount of the human iPSC-derived CD4+ T cell of any one of [8] to [10].
[17] The human iPSC-derived CD4+ T cell of any one of [8] to [10] for use in treating cancer or a viral infectious disease.
[18] Use of the human iPSC-derived CD4+ T cell of any one of [8] to [10] for the production of a therapeutic agent for cancer or a viral infectious disease.

### [Advantageous Effects of Invention]

According to the present invention, it is possible to stably produce CD4+ T cells, particularly CD4 SP T cells, from peripheral blood T cells via iPSCs without introducing the CD4 gene. This allows efficient production of CD4+ T cells that can be used, for example, in cancer immunotherapy. Furthermore, the CD4+ T cells obtained according to the present invention possess both the Th1 cell phenotype and antigen-specific cytotoxic activity, and are capable of killing target cells (e.g., cancer cells, virus-infected cells) by themselves and activating target cell-specific CTLs. Therefore, the therapeutic effect can be further enhanced by using them in combination with target cell-specific CTLs or CAR-T cells.

### [Brief Description of Drawings]

[Fig. 1]

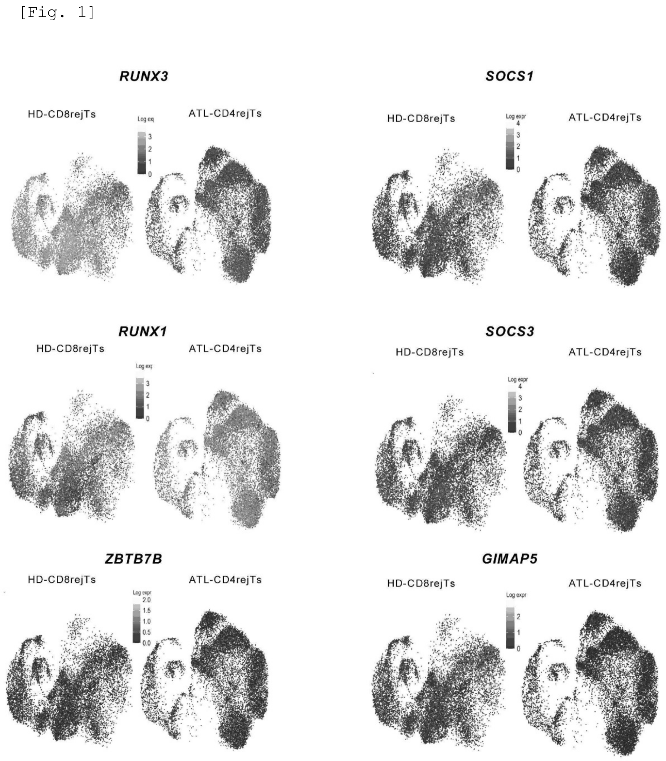

   Fig. 1 shows a comparison of the expression of RUNX3, RUNX1, ZBTB7B, SOCS1, SOCS3, and GIMAP5 between CD4T-iPSC-derived CD8+ T cells (HD-CD8rejTs) and ATL-iPSC-derived CD4+ T cells (ATL-CD4rejTs).
[Fig. 2]

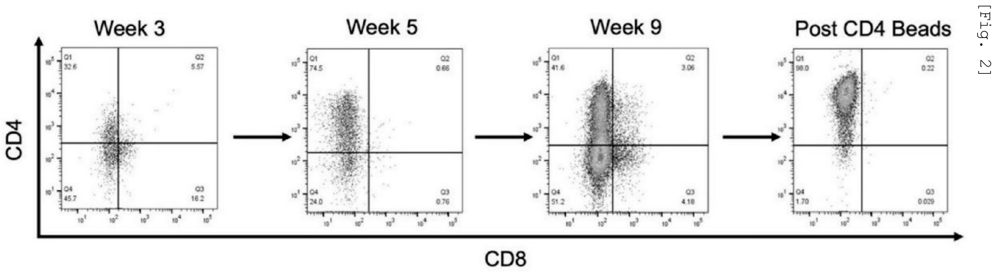

   Fig. 2 shows the time-course changes in the CD4/CD8 ratio as determined by flow cytometry during induction of differentiation of RUNX3 KO CD4T-iPSC-derived hematopoietic progenitor cells.
[Fig. 3]

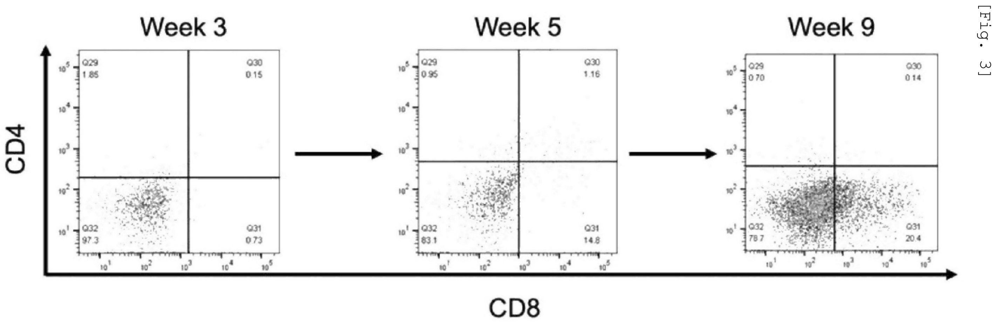

   Fig. 3 shows the time-course changes in the CD4/CD8 ratio as determined by flow cytometry during induction of differentiation of RUNX3 WT CD4T-iPSC-derived hematopoietic progenitor cells.
[Fig. 4]

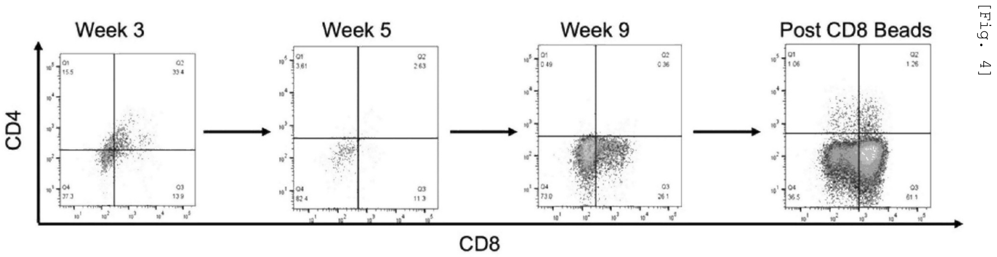

   Fig. 4 shows the time-course changes in the CD4/CD8 ratio as determined by flow cytometry during induction of differentiation of RUNX3 Overexpression ATL-iPSC-derived hematopoietic progenitor cells.
[Fig. 5]

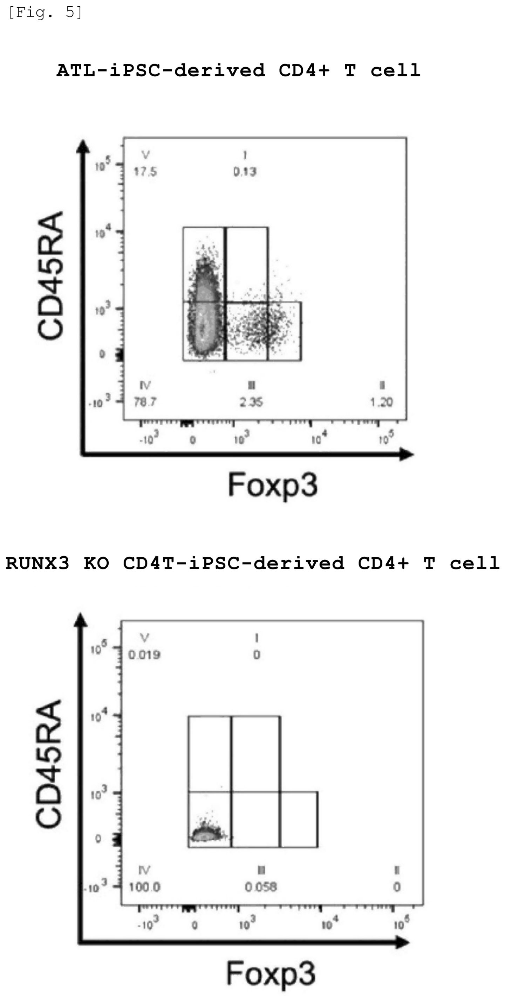

   Fig. 5 shows the results of flow cytometry analysis using anti-CD4, CD45RA, and Foxp3 antibodies, of CD4+ T cells derived from RUNX3 KO CD4T-iPSCs.
[Fig. 6]

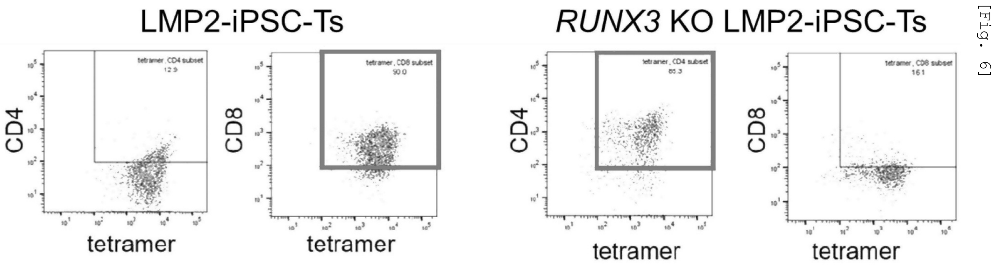

   Fig. 6 shows that CD8SP T cells (left) induced to differentiate from LMP2-specific CTL-derived iPSCs and CD4SP T cells (right) induced to differentiate from the same iPSCs after RUNX3 knockout maintain class I-restricted LMP2 antigen specificity.
[Fig. 7]

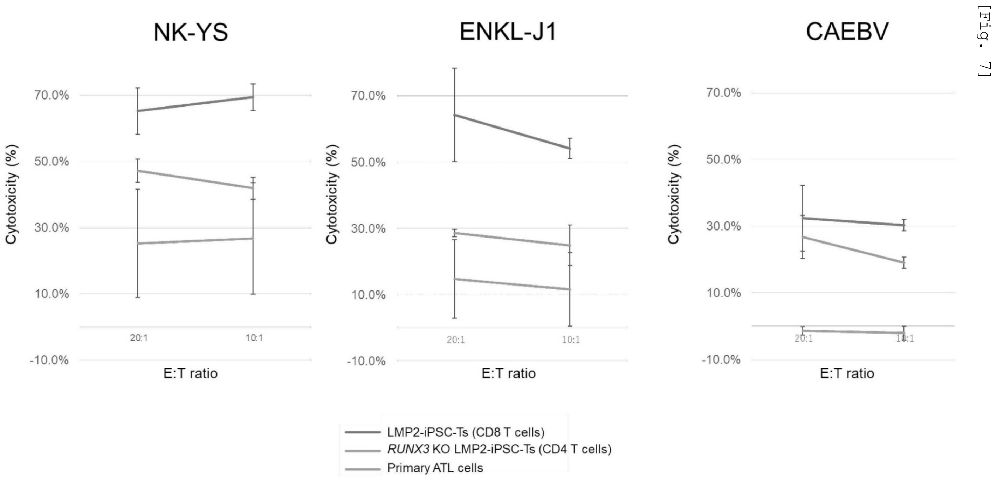

   Fig. 7 shows that RUNX3 KO LMP2-iPSC-Ts have antigen-specific cytotoxic activity, although they are inferior to that of LMP2-iPSC-Ts. The line plots in each graph show LM2-iPSC-Ts (CD8+), RUNX3 KO LMP2-iPSC-Ts, and primary ATL cells (control), from the top. The effector cell:target cell (E:T) ratios are 20:1 and 10:1 from the left.
[Fig. 8A]

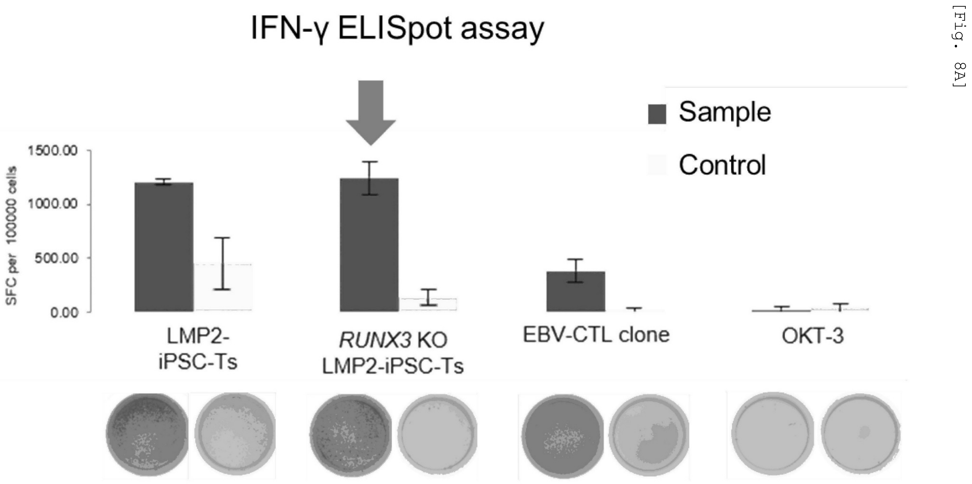

   Fig. 8A shows that RUNX3 KO LMP2-iPSC-Ts abundantly secrete IFNγ in response to antigen stimulation.
[Fig. 8B]

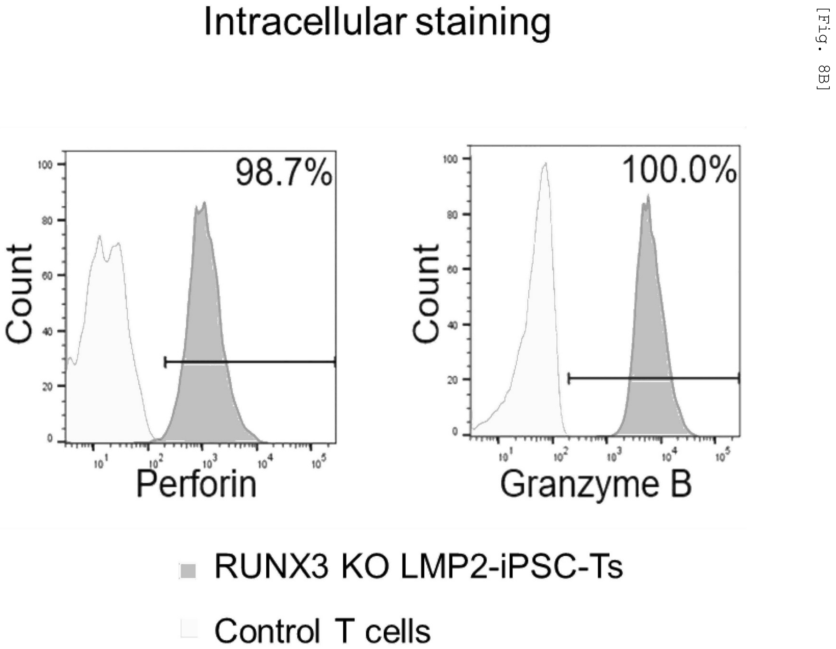

   Fig. 8B shows that RUNX3 KO LMP2-iPSC-Ts abundantly produce perforin and granzyme B in response to antigen stimulation.
[Fig. 9]

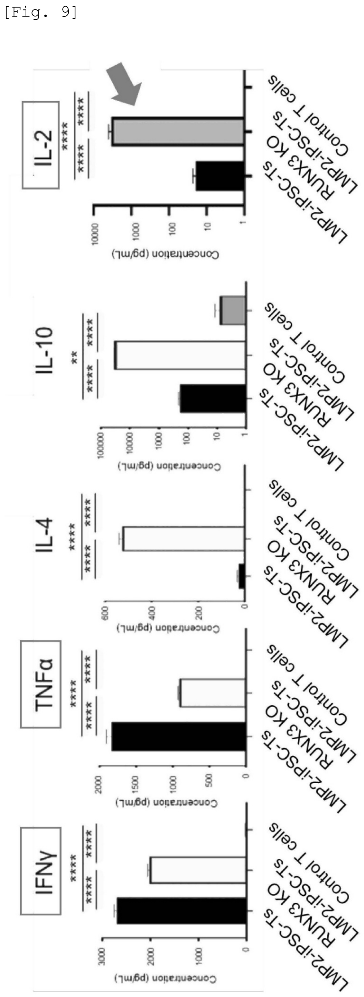

   Fig. 9 shows that RUNX3 KO LMP2-iPSC-Ts highly secrete various cytokines in response to antigen stimulation. **: p<0.01, ****: p<0.0001
[Fig. 10A]

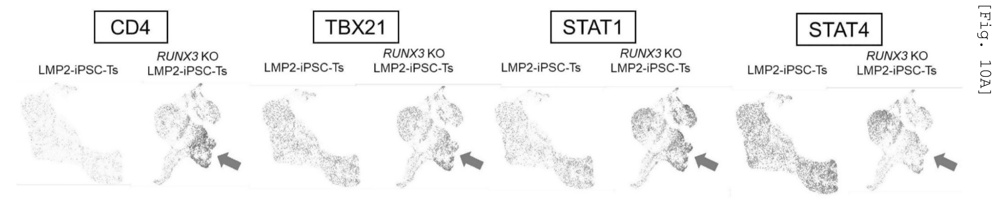

   Fig. 10A shows that RUNX3 KO LMP2-iPSC-Ts highly express TBX21, STAT1, and STAT4.
[Fig. 10B]

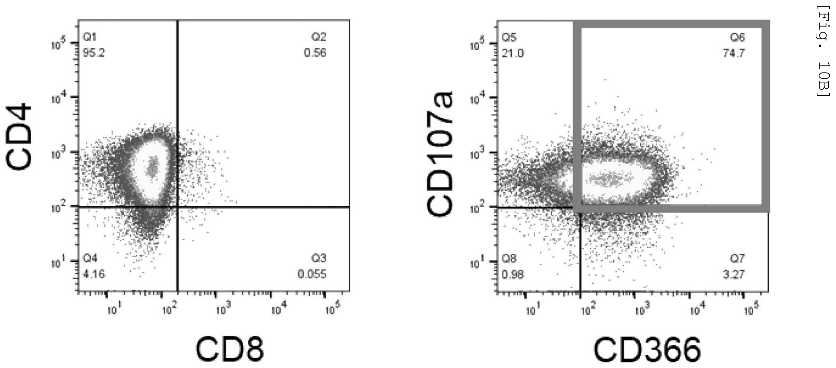

   Fig. 10B shows that RUNX3 KO LMP2-iPSC-Ts are CD107a and CD366 positive.
[Fig. 11]

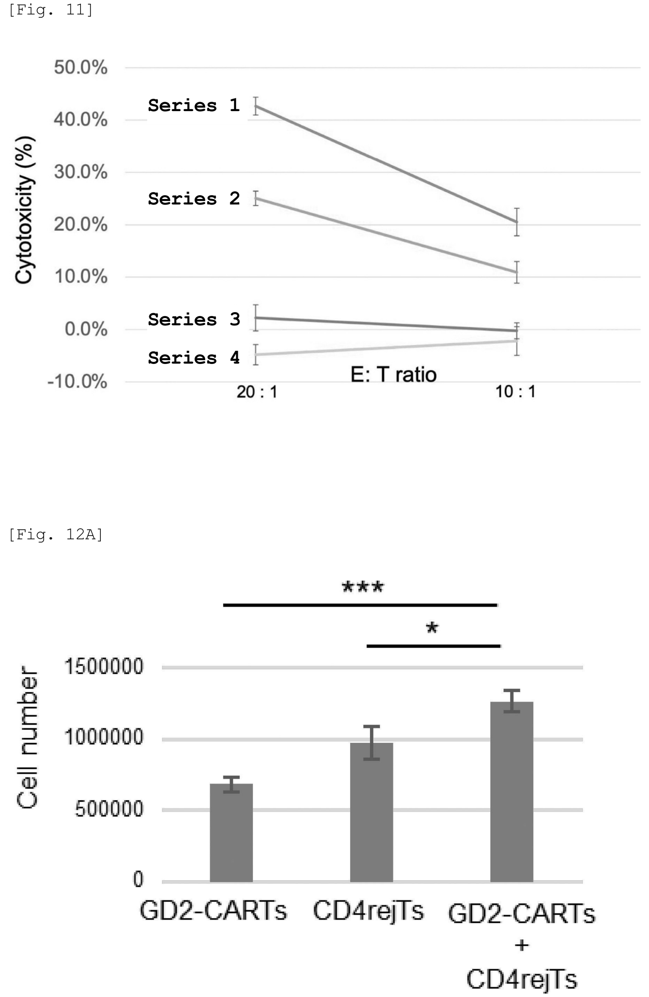

   Fig. 11 shows that when CD8+ T cells induced to differentiate from HPV16E6-specific CTL-derived iPSCs are co-cultured with CD4+ T cells induced to differentiate from the iPSCs after RUNX3 knockout, the cytotoxic activity of HPV16E6-iPSC-Ts is enhanced. Series 1: HPV16E6-iPSC-Ts + RUNX3 KO HPV16E6-iPSC-Ts (vs. antigen-positive cells), Series 2: HPV16E6-iPSC-Ts (vs. antigen-positive cells), Series 3: HPV16E6-iPSC-Ts + RUNX3 KO HPV16E6-iPSC-Ts (vs. antigen-negative cells), Series 4: HPV16E6-iPSC-Ts (vs. antigen-negative cells). The E:T ratios are 20:1 and 10:1 from the left. (Series 1, 2, 3, and 4 are shown from the top)
[Fig. 12A]
   Fig. 12A shows that co-culturing GD2-specific CAR-T cells with RUNX3 KO T-iPSC-derived CD4+ T cells promotes cell proliferation.
[Fig. 12B]

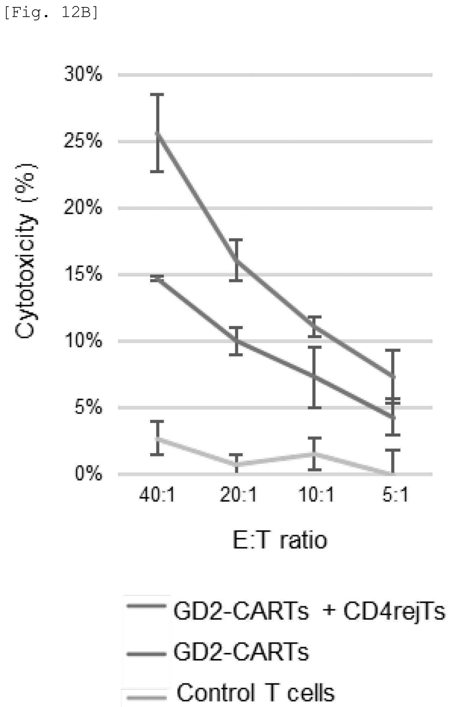

   Fig. 12B shows that co-culturing GD2-specific CAR-T cells with RUNX3 KO T-iPSC-derived CD4+ T cells enhances cytotoxic activity against GD2-positive EBV-associated lymphoma cells. The line plots show GD2-CARTs + CD4rej Ts, GD2-CARTs, and primary ATL cells (control), from the top.
[Fig. 13]

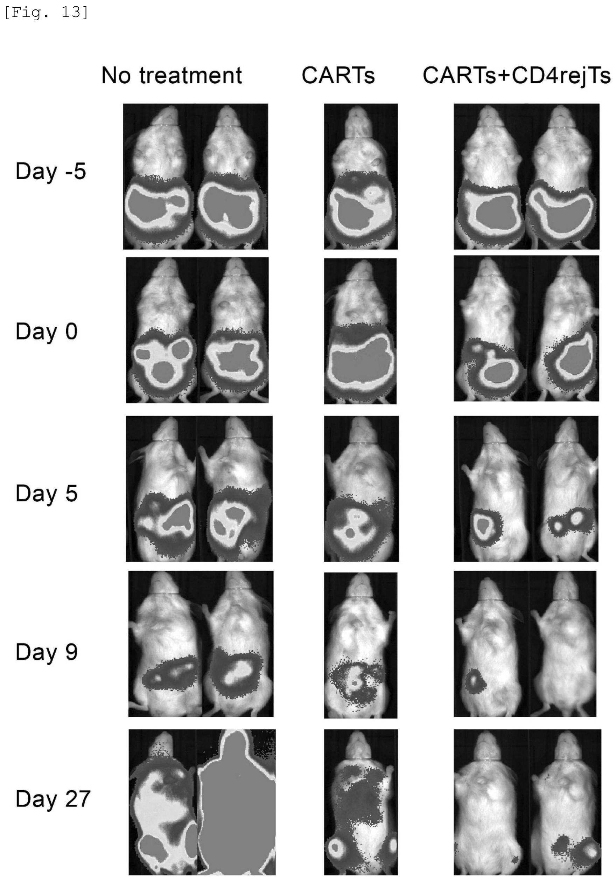

   Fig. 13 shows that when GD2-specific CAR-T cells (CARTs) and RUNX3 KO T-iPSC-derived CD4+ T cells (CD4rejTs) are co-cultured and administered to mice transplanted with GD2-positive EBV-associated lymphoma cells, the tumor growth-inhibitory effect is more enhanced than administration of CAR-T cells alone.

### [Description of Embodiments]

The present invention provides a method for producing a non-regulatory CD4+ T cell via a pluripotent stem cell (hereinafter also to be referred to as "the production method of CD4+ T cell of the present invention", "the production method of the present invention"). One embodiment of the production method of CD4+ T cell of the present invention includes a step of knocking out one or more genes selected from RUNX3, ZBTB7B, SOCS1, SOCS3, and GIMAP5 and/or overexpressing the RUNX1 gene in an iPSC induced from a human-derived somatic cell. In another embodiment, the production method of the present invention includes, before the aforementioned step (step (2)), a step of inducing an iPSC from a human-derived somatic cell (step (1)).

In the following, step (1) and step (2) are described.

### (1-1) Isolation of human-derived somatic cell

The human iPSC used in the production method of the present invention can be induced from any human somatic cell. Examples of the human somatic cell include tissue stem cells (somatic stem cells) such as hematopoietic stem cells, mesenchymal stem cells, neural stem cells, and dental pulp stem cells; tissue progenitor cells; differentiated cells such as lymphocytes (e.g., peripheral blood mononuclear cells, peripheral blood-derived T cells, tumor-infiltrating lymphocytes, and umbilical cord blood-derived mononuclear cells), fibroblasts (skin cells, etc.), epithelial cells, endothelial cells, muscle cells, hair cells, liver cells, gastric mucosal cells, intestinal cells, spleen cells, pancreatic cells (exocrine pancreatic cells, etc.), brain cells, lung cells, kidney cells, and adipocytes. Preferred examples include T cells (e.g., antigen-specific T cells, peripheral blood-derived T cells), peripheral blood mononuclear cells, fibroblasts, and the like. In the following, antigen-specific T cells or peripheral blood-derived T cells are used as examples and described in detail, but those skilled in the art can appropriately collect other human somatic cells by conventional methods.

The human-derived antigen-specific T cells or peripheral blood-derived T cells to be used may be those from a healthy subject or a patient with a viral infectious disease which is rich in T cells. Tissues, peripheral blood, and the like from a patient with a viral infectious disease or a healthy individual who has previously been infected with a virus are preferred.

The following describes the isolation of antigen-specific T cells or peripheral blood-derived T cells from a patient with a viral infection as an example. T cells from a patient with a viral infection can be isolated by a known method, for example, from the tissue of an ATL patient. Tissues derived from ATL patients include tissues containing T cells, such as peripheral blood, lymph nodes, bone marrow, thymus, spleen, umbilical cord blood, and diseased tissue. Among these, peripheral blood is preferred because it is less invasive to human and easy to prepare.

In one preferred embodiment, human peripheral blood-derived T cells can be isolated from the peripheral blood of the patient to be treated (e.g., cancer patient, virus-infected individual). When using peripheral blood from a person other than the patient to be treated, the patient HLA-restricted peripheral blood, namely peripheral blood from an individual whose HLA antigens match those of the patient to be treated can be used. However, CD4+ T cells can also be produced in other cases.

Antigen-specific T cells are not particularly limited as long as they are T cells in which the T cell receptor (TCR) has been rearranged so that they specifically bind to a particular antigen. When the obtained CD4+ T cells are intended to be used as an immunotherapeutic agent, the antigen is preferably a disease-specific antigen peptide, such as a cancer antigen peptide, a viral antigen peptide, and the like. Antigen-specific T cells can be obtained, for example, by isolating lymphocytes that have infiltrated the lesion site (e.g., tumor, virus-infected tissue) of a patient, or by sensitizing T cells isolated from peripheral blood to an antigen or by introducing an antigen-specific TCR gene or chimeric antigen receptor (CAR) gene to confer the desired antigen specificity.

Known methods for isolating antigen-specific T cells or peripheral blood-derived T cells include, for example, magnetic selection using magnetic beads for cell separation, and flow cytometry using a cell sorter and an antibody against a cell surface antigen, such as an anti-CD4 antibody or an anti-CD8 antibody.

Antigen-specific T cells or peripheral blood-derived T cells may be CD8+ T cells or CD4+ T cells. Regardless of which starting material is used, the production method of the present invention can produce CD4+ T cells, particularly CD4SP cells. However, when CD8+ T cells are used as the starting material, the CD4+ T cells obtained maintain class I-restricted antigen specificity even though they do not express coreceptor CD8 molecules. On the other hand, when CD4+ T cells are used as the starting material, the CD4+ T cells obtained maintain class II-restricted antigen specificity.

### (1-2) Establishment of iPSC from human somatic cell

Isolated human somatic cell is reprogrammed to become iPSC.

As used herein, "iPSC" is a cell also known as artificial pluripotent stem cell or induced pluripotent stem cell, and can be induced by introducing cell reprogramming factor(s) into a somatic cell such as an antigen-specific T cell, peripheral blood-derived T cell, peripheral blood mononuclear cell, or fibroblast.

The "cell reprogramming factor" is not particularly limited as long as it is a factor that can confer differentiation pluripotency to a somatic cell, either alone or in cooperation with other pluripotency factors, when introduced into the aforementioned somatic cell, such as antigen-specific T cell, peripheral blood-derived T cell, peripheral blood mononuclear cell, or fibroblast. It is preferably at least one type of protein selected from the group consisting of Oct3/4, c-Myc, Sox2, Klf4, Klf5, LIN28, Nanog, ECAT1, ESG1, Fbx15, ERas, ECAT7, ECAT8, Gdf3, Sox15, ECAT15-1, ECAT15-2, Fthl17, Sal14, Rex1, Utf1, Tcl1, Stella, β-catenin, Stat3, and Grb2. From the viewpoint of efficient establishment of iPSC with fewer factors among these proteins, it is more preferable to introduce the four factors of Oct3/4, Sox2, Klf4, and c-Myc into the aforementioned antigen-specific T cell or peripheral blood-derived T cell. When using the CD4+ T cells obtained by the present invention as a medicament, such as a human immunotherapeutic agent and the like, it is also preferable to introduce the three factors excluding c-Myc or to use L-Myc instead of c-Myc, in order to reduce the risk of tumorigenesis. Other preferable combinations of reprogramming factors are described in, for example, WO2007/069666, WO2008/118820, WO2009/007852, WO2009/032194, WO2009/058413, WO2009/057831, WO2009/075119, WO2009/079007, WO2009/091659, WO2009/101084, WO2009/101407, WO2009/102983, WO2009/114949, WO2009/117439, WO2009/126250, WO2009/126251, WO2009/126655, WO2009/157593, WO2010/009015, WO2010/033906, WO2010/033920, WO2010/042800, WO2010/050626, WO 2010/056831, WO2010/068955, WO2010/098419, WO2010/102267, WO 2010/111409, WO 2010/111422, WO2010/115050, WO2010/124290, WO2010/147395, WO2010/147612, Huangfu D, et al. (2008), Nat. Biotechnol., 26: 795-797, Shi Y, et al. (2008), Cell Stem Cell, 2: 525-528, Eminli S, et al. (2008), Stem Cells. 26:2467-2474, Huangfu D, et al. (2008), Nat Biotechnol. 26:1269-1275, Shi Y, et al. (2008), Cell Stem Cell, 3, 568-574, Zhao Y, et al. (2008), Cell Stem Cell, 3:475-479, Marson A, (2008), Cell Stem Cell, 3, 132-135, Feng B, et al. (2009), Nat Cell Biol. 11:197-203, R.L. Judson et al., (2009), Nat. Biotech., 27:459-461, Lyssiotis CA, et al. (2009), Proc Natl Acad Sci U S A. 106:8912-8917, Kim JB, et al. (2009), Nature. 461:649-643, Ichida JK, et al. (2009), Cell Stem Cell. 5:491-503, Heng JC, et al. (2010), Cell Stem Cell. 6:167-74, Han J, et al. (2010), Nature. 463:1096-100, Mali P, et al. (2010), Stem Cells. 28:713-720, Maekawa M, et al. (2011), Nature. 474:225-9., and the like.

In the present invention, the method for introducing the aforementioned cell reprogramming factors into the aforementioned human somatic cell, such as antigen-specific T cell, peripheral blood-derived T cell, peripheral blood mononuclear cell, or fibroblast is not particularly limited. They can be introduced in the form of proteins or nucleic acids and using known methods as appropriate. For example, when introducing in the form of a nucleic acid encoding the aforementioned cell reprogramming factor into the aforementioned human somatic cell such as antigen-specific T cell, peripheral blood-derived T cell, peripheral blood mononuclear cell, or fibroblast, the nucleic acid (e.g., cDNA, RNA) is inserted into an appropriate expression vector (e.g., a viral vector, a non-viral vector) containing a promoter that functions in the somatic cell, and the expression vector can be introduced into the cell by viral infection, lipofection method, liposome method, electroporation method, calcium phosphate coprecipitation method, DEAE-dextran method, microinjection method, or the like.

Examples of such expression vector include viral vectors such as lentivirus, retrovirus, adenovirus, adeno-associated virus, herpes virus, and Sendai virus, and animal cell expression plasmids. From the viewpoints of low incidence of insertional mutagenesis, high gene transfer efficiency, and a large number of copies of the introduced gene, it is preferable to use Sendai virus to introduce the aforementioned nucleic acid encoding the cell reprogramming factor into the human somatic cell.

Examples of promoters used in such expression vectors include SRα promoter, SV40 promoter, LTR promoter, CMV promoter, RSV promoter, and HSV-TK promoter. Furthermore, such promoters may be capable of controlling the expression of a gene inserted downstream of the promoter, depending on the presence or absence of a drug (e.g., tetracycline). In addition to the promoter, the expression vector may also contain an enhancer, a poly(A) addition signal, a selection marker gene (e.g., a neomycin resistance gene), an SV40 replication origin, and the like.

When introducing such reprogramming factor, it is preferable to express the SV40 large T antigen along with each reprogramming factor in the target cell, since this increases the proliferation and survival rate of the cell required for reprogramming.

Therefore, the optimal embodiment for introducing cell reprogramming factors into the aforementioned antigen-specific T cell or peripheral blood-derived T cell is to infect the aforementioned antigen-specific T cell or peripheral blood-derived T cell with a Sendai virus vector into which nucleic acids encoding the four factors Oct3/4, Sox2, Klf4, and c-Myc have been inserted, and a Sendai virus vector into which nucleic acid encoding SV40 large T antigen has been inserted. The iPSC may also be established using a Sendai virus vector containing additional factors, or without using a Sendai virus vector into which nucleic acid encoding SV40 large T antigen has been inserted. As a vector other than Sendai virus, episomal vectors such as oriP/EBNA-1 and the like are preferably used.

Alternatively, the reprogramming factor may be introduced in the form of mRNA, in which case the introduction can be performed using well-known and conventional techniques for mRNA vaccines, etc. (nucleic acid modification, introduction via lipid nanoparticles).

Examples of the medium for culturing the aforementioned antigen-specific T cells or peripheral blood-derived T cells include known media suitable for culturing T cells (more specifically, Roswell Park Memorial Institute (RPMI) 1640 medium, AIM V^{™} medium, or NS-A2, which contains other cytokines and human serum) can be used. The medium may also contain amino acids (e.g., L-glutamine) and antibiotics (e.g., streptomycin and penicillin) necessary for culturing.

No particular limitations are imposed on the conditions when or after introducing the aforementioned cell reprogramming factors and the like into the aforementioned antigen-specific T cell or peripheral blood-derived T cell. It is preferable to culture the aforementioned antigen-specific T cells or peripheral blood-derived T cells after introduction of the aforementioned factors under feeder-free conditions. For example, wells coated with a solution of iMatrix-511, which is a laminin 511 E8 fragment, or vitronectin or matrigel can be mentioned. The iPSC can also be established by culture under feeder cell conditions. Examples of the feeder cell include mouse embryonic fibroblasts (MEFs), STO cells, and SNL cells whose cell division has been halted by radiation exposure or antibiotic treatment.

Furthermore, during the process of inducing T-iPSC from the aforementioned antigen-specific T cell or peripheral blood-derived T cell, it is preferable to add an iPSC medium starting from the next day of the induction. Thereafter, it is preferable to gradually replace the CTL medium with iPS medium by exchanging half of the medium every other day.

Furthermore, it is preferable to culture cells while gradually replacing a known medium suitable for culturing T cell with a medium suitable for culturing iPSC, during transition from the aforementioned antigen-specific T cell or peripheral blood-derived T cell to iPSC. As a medium suitable for such iPSC culture, a known medium can be appropriately selected and used. For example, StemFit AK03N is preferred for iMatrix coating, Essential 8 Medium is preferred for vitronectin coating, mTeSR is preferred for Matrigel, and Dulbecco's modified Eagle's medium/F12 medium (human iPSC medium) containing Knockout Serum Replacement, L-glutamine, non-essential amino acids, 2-mercaptoethanol, b-FGF, etc. is preferred on feeder cells such as MEF cells.

T-iPSC induced from the aforementioned antigen-specific T cell or peripheral blood-derived T cell in this manner can be selected by appropriately selecting known methods. Examples of such known methods include a method in which iPSC-like colonies are selected by observing the morphology thereof under a microscope. Alternatively, all established colonies may be passaged as they are without selecting each T-iPSC colony.

That the selected cells are T-iPSCs can be confirmed, for example, by a method of detecting the expression of undifferentiated cell-specific markers (SSEA-4, Tra-1-60, Tra-1-81, etc.) in the selected cells by q-PCR or ALP staining, or by transplanting the selected cells into mice and observing teratoma formation.

The timing for selecting and recovering these cells can be determined appropriately while observing the growth state of the colonies. Generally, it is 10 to 40 days, preferably 14 to 28 days, after introducing the aforementioned cell reprogramming factor into the aforementioned antigen-specific T cell or peripheral blood-derived T cell. Unless otherwise specified in the above, the culture environment is preferably 5% CO₂, 35 - 38°C, more preferably 37°C. Efficient growth of colony can also be observed in a hypoxic culture environment (oxygen concentration: e.g., 5%).

### (2) Operation of gene expression from established T-iPSC

In this step, target genes (RUNX3, ZBTB7B, SOCS1, SOCS3, and/or GIMAP5) are knocked out and/or RUNX1 is overexpressed in the established T-iPSC. Involvement of these target genes in CD4/CD8 lineage selection has been suggested. As described in the below-mentioned Examples, the inventors have found for the first time that the expression of these genes (RUNX3, ZBTB7B, SOCS1, SOCS3, and/or GIMAP5) decreased and the expression of RUNX1 increased in ATL-iPSC-derived CD4+ T cells, compared to CD8+ T cells obtained by inducing differentiation from normal T-iPSCs.

The target gene to be knocked out is not particularly limited as long as it is one or more of the above-mentioned five genes, and preferably includes RUNX3. These genes can be knocked out or RUNX1 can be overexpressed by gene transfer techniques using viral vectors containing known target genes or genome editing techniques, and genome editing techniques are preferred. To avoid the risk associated with insertional mutation, overexpression of the RUNX1 gene can be performed, for example, by knocking in the RUNX1 gene under the control of a promoter capable of constitutive expression or a T cell-specific promoter at the endogenous RUNX1 locus in iPSC, or by introducing the RUNX1 gene by using a viral vector such as Sendai virus, lentivirus, or retrovirus, or another episomal vector. Preferably, the RUNX1 gene is knocked in.

Genome editing techniques include ZFN, TALEN, and CRISPR-Cas9, and CRISPR-Cas9 is more preferred. CRISPR-Cas9 consists of a guide RNA (gRNA) that specifically recognizes and binds to the target DNA sequence and nuclease Cas9. The double-stranded DNA is cleaved by Cas9 guided by the gRNA, and the target gene is knocked out when the cleaved double-stranded DNA is repaired. Appropriate candidate gRNA sequence can be designed based on the sequence information of each target gene (provided in various public databases, such as NCBI, and easily accessible to those skilled in the art). Various commercially available tools (e.g., Chop Chop Harvard, CRISPR Design, etc.) can also be used as gRNA design tools. RUNX1 gene can be knocked in by introducing a donor DNA containing the RUNX1 gene under the control of a promoter capable of constitutive expression or a T cell-specific promoter into iPSC together with gRNA.

Genome-edited T-iPSC is cultured as a single cell. Genomic DNA is extracted from the obtained single colonies and subjected to genotyping PCR. The PCR products are purified and Sanger sequencing is performed to confirm that the genomic DNA from each single-cell colony has acquired a frameshift mutation, resulting in generation of an amino acid sequence completely different from the wild-type after the insertion/deletion, and further that a stop codon has been acquired on the way thereof.

The state of TCR gene rearrangement in the cell in which the target gene has been knocked out or knocked in can be detected by genomic PCR, whereby it can be confirmed that the cell from which the T-iPSC was derived is an antigen-specific T cell.

The thus-obtained genetically modified iPSCs can be induced to differentiate into T cells by a method known per se, for example, the methods described in Patent Literatures 1 to 4. For example, when inducing T cells from iPSCs, the cells may be activated by stimulation with anti-CD3 antibody and anti-CD28 antibody in the presence of interleukin-2 (IL-2), interleukin-7 (IL-7), or interleukin-15 (IL-15). Alternatively, the cells may be activated by stimulation with at least one substance selected from the group consisting of phytohemagglutinin (PHA), interleukin-2 (IL-2), alloantigen-expressing cell, anti-CD3 antibody, anti-CD28 antibody, CD3 agonist, and CD28 agonist. Such stimulation can be performed, for example, by adding PHA, IL-2, anti-CD3 antibody, and/or anti-CD28 antibody to the medium and culturing, for a certain period of time, the cells undergoing differentiation induction. The anti-CD3 antibody and/or anti-CD28 antibody may be bound to magnetic beads, etc., and the cells may be stimulated by culturing them for a certain period of time on a culture dish with anti-CD28 antibody and/or anti-CD28 antibody bound to the surface, instead of adding these antibodies to the medium.

More specifically, for example, the method described in Reference Example 1 (3) below is preferred, but the method is not limited thereto.

The period of differentiation-inducing culture from iPSCs to T cells is not particularly limited as long as a sufficient CD4+ fraction is obtained, and is, for example, 3 weeks or more, preferably 5 weeks or more. The upper limit of the culture period is not particularly limited, and can be, for example, 15 weeks or less, preferably 12 weeks or less. The increase in the proportion of the CD4+ T cell population, preferably the CD4SP T cell population, can be confirmed, for example, by collecting a portion of the cells during differentiation-inducing culture and performing flow cytometry using an anti-CD4 antibody, preferably further an anti-CD8 antibody.

The thus-obtained CD4+ T cells can be isolated using an appropriately selected known method. Examples of such known method include flow cytometry using an antibody against a cell surface marker such as CD4 and a cell sorter.

That the obtained CD4+ T cells are not non-regulatory can be confirmed by analyzing the expression of surface antigen markers characteristic of regulatory T cells, such as Foxp3, by flow cytometry using antibodies against them.

In addition, the present inventors confirmed that when RUNX3 is forcibly expressed in ATL-iPSCs, the RUNX3-overexpressing cells induced to differentiate do not differentiate into CD4+ T cells, and demonstrated that the expression level of RUNX3 determines the induction of differentiation into CD4/CD8 T cells. Similarly, the induction of differentiation into CD4/CD8 T cells can be controlled by regulating the expression of ZBTB7B, SOCS1, SOCS3, and GIMAP5, which were identified to show reduced expression in ATL-iPSC-CD4+ T cells, and RUNX1 which was identified to show increased expression in ATL-iPSC-CD4+ T cells, as a result of differential expression analysis of ATL-iPSC-CD4+ T cells and CD4T-iPSC-CD8+ T cells.

The T-iPSC-derived CD4+ T cells obtained by the production method of the present invention may be any as long as they are induced by any of the methods described above and are CD4 positive, particularly CD4 single-positive (SP). Preferably, they have the following properties (a) to (e):
(a) being CD4 single-positive;
(b) having a TCR specific to an antigen peptide presented on a cancer cell or a virus-infected cell;
(c) exhibiting one or more of the following Th1 cell phenotypes:
   (c1) highly secreting IFNγ and IL-2;
   (c2) highly expressing one or more genes selected from the group consisting of TBX21, STAT1, and STAT4;
   (c3) expressing CD107a and CD366;
(d) independently having cytotoxic activity specific to the aforementioned antigen peptide of (b); and
(e) enhancing the proliferation and/or cytotoxic activity of cancer antigen- or viral antigen-specific CTL or CAR-T cell.

When the original antigen-specific T cell is a CD8+ T cell, the rearranged TCR is inherited even through reprogramming, and therefore, CD4+ T cells obtained via iPSCs are class I-restricted. Since CD4SP T cells do not express CD8 molecules, reduction of the TCR binding ability to antigen peptide-class I molecule complex and reduction of the activation ability due to the absence of CD8 stimulation are expected. However, surprisingly, the obtained CD4SP T cells abundantly produce cytotoxic cytokines such as IFNγ, TNFα, perforin, and granzyme B, and retain the original antigen-specific cytotoxic activity.

Moreover, since the CD4SP T cells abundantly secrete cytokines and function as helper T cells, activate antigen-presenting cells, and can activate CD8+ T cells (CTLs), they can further enhance the cytotoxic activity of CTLs. The CD4SP T cells abundantly secrete Th1 cytokines such as IFNγ and IL-2, and also show Th1 cell phenotype from the expression profiles of transcription factors and surface antigens. Therefore, they are expected to function as Th1 cells, for example, by activating target cells such as macrophage, CTL, and NK cell, thereby achieving the effects of infection protection and anti-tumor immunity. One of the reasons why cancer immunotherapy using peripheral blood CTLs is not as effective as expected may be that CD8+ CTLs with low IL-2 productivity die before they can kill cancer cells. However, the CD4SP cells obtained by the present invention have high IL-2 secretion ability, and are expected to promote the survival and proliferation of CTLs and further enhance the cytotoxic activity thereof. Furthermore, Th1 cell therapy is considered to be able to suppress the increase and accumulation of regulatory T cells, which are induced in tumors and regional lymph nodes as cancer grows, in an IFNγ-dependent manner, and can suppress cancer immune escape.

In a preferred embodiment, the CD4SP cells obtained by the present invention highly secrete cytokines such as IL-4 and IL-10, in addition to Th1 cytokine.

iPSC-derived CD4SP T cells having the above-mentioned characteristics are not conventionally known and are novel T cells. Therefore, the present invention also provides human iPSC-derived T cells having the following properties:
(a) being CD4 single-positive;
(b) having a TCR specific to an antigen peptide presented on a cancer cell or a virus-infected cell;
(c) exhibiting one or more of the following Th1 cell phenotypes:
   (c1) highly secreting IFNγ and IL-2;
   (c2) highly expressing one or more genes selected from the group consisting of TBX21, STAT1, and STAT4;
   (c3) expressing CD107a and CD366;
(d) independently having cytotoxic activity specific to the aforementioned antigen peptide of (b); and
(e) enhancing the proliferation and/or cytotoxic activity of cancer antigen- or viral antigen-specific CTL or CAR-T cell (hereinafter also to be referred to as "the CD4+ T cell of the present invention").

The CD4+ T cells of the present invention may be obtained by any method as long as they have the above-mentioned properties. Preferably, the expression of one or more genes selected from RUNX3, ZBTB7B, SOCS1, SOCS3, and GIMAP5 is suppressed and/or the expression of the RUNX1 gene is enhanced in the T cells. Preferably, the expression of at least the RUNX3 gene is suppressed in the CD4+ T cells of the present invention. No particular limitation is imposed on the form of suppression of the expression of these genes, but preferably, the genes are knocked out or overexpressed (preferably knocked in) by genetic manipulation.

It has been confirmed that the CD4+ T cells obtained by the production method of the present invention are not regulatory T cells. It has also been confirmed that the CD4+ T cells exhibit a Th1 cell phenotype. Therefore, the CD4+ T cells of the present invention, including the CD4+ T cells obtained by the production method of the present invention, can be used, for example, in various cancer immunotherapies and immunotherapy for infectious diseases, preferably viral infectious diseases. Therefore, the present invention provides a pharmaceutical composition, preferably an immunotherapeutic agent, containing CD4+ T cells obtained by the production method of the present invention or the CD4+ T cells of the present invention.

The pharmaceutical composition of the present invention can be a prophylactic or therapeutic drug for cancer or infectious diseases (e.g., viral infectious diseases). The cancer to which the pharmaceutical composition can be applied is not particularly limited and examples include, but are not limited to, acute lymphatic leukemia, alveolar rhabdomyosarcoma, bladder cancer, bone cancer, brain cancer (e.g., medulloblastoma), breast cancer, cancer of the anus, anal canal, or anorectal region, eye cancer, intrahepatic bile duct cancer, joint cancer, cancer of the neck, gallbladder, or pleura, cancer of the nose, nasal cavity, or middle ear, oral cancer, vulvar cancer, chronic myeloid leukemia, colon cancer, esophageal cancer, cervical cancer, fibrosarcoma, gastrointestinal carcinoid tumor, head and neck cancer (e.g., head and neck squamous cell carcinoma), hypopharyngeal cancer, kidney cancer, laryngeal cancer, leukemia (e.g., acute lymphoblastic leukemia, acute lymphocytic leukemia, chronic lymphocytic leukemia, acute myeloid leukemia), humoral tumor, liver cancer, lung cancer (e.g., non-small cell lung cancer), lymphoma (e.g., Hodgkin lymphoma, non-Hodgkin lymphoma, diffuse large B-cell lymphoma, follicular lymphoma), malignant mesothelioma, mast cell tumor, melanoma, multiple myeloma, nasopharyngeal cancer, ovarian cancer, pancreatic cancer; cancer of the peritoneum, omentum and mesentery; pharyngeal cancer, prostate cancer, rectal cancer, kidney cancer, skin cancer, small intestine cancer, soft tissue cancer, solid tumor, stomach cancer, testicular cancer, thyroid cancer, ureteral cancer, and the like.

Furthermore, the infectious diseases to which the pharmaceutical composition is applied is also not particularly limited, and examples thereof include viral infections (e.g., RNA virus, DNA virus, human immunodeficiency virus (HIV), hepatitis A, B, and C viruses, herpes simplex virus (HSV), cytomegalovirus (CMV), Epstein-Barr virus (EBV), human papillomavirus (HPV)), parasitic infections (e.g., protozoan and metazoan pathogens such as Plasmodium species, Leishmania species, Schistosoma species, and Trypanosoma species), bacterial infections (e.g., Mycobacteria, particularly M. tuberculosis, Salmonella, Streptococci, E. coli, Staphylococci), fungal infections (e.g., Candida species, Aspergillus species, Pneumocystis carinii), and the like.

The pharmaceutical composition of the present invention can be used in combination with cancer antigen- or viral antigen-specific CTLs or CAR-T cells. The CD4+ T cells of the present invention exhibit cytotoxic activity specific to the antigen recognized by the TCR of the original T cell, and can proliferate and activate other CTLs and CAR-T cells, thereby enhancing the cytotoxic activity thereof. Therefore, the combined use of the two can achieve a higher therapeutic effect than the administration of CTLs or CAR-T cells alone.

The pharmaceutical composition of the present invention can be used in combination with antigen-presenting cells (e.g., dendritic cells) and/or antigenic peptides presented on cancer cells or virus-infected cells. The CD4+ T cells of the present invention can activate antigen-presenting cells and activate CTLs specific to the antigenic peptide. Therefore, the combined use of the two can efficiently induce cancer antigen- or viral antigen-specific CTLs in the subject of administration.

The pharmaceutical composition of the present invention can be prepared by formulating the CD4+ T cells obtained by the production method of the present invention or the CD4+ T cells of the present invention using known pharmaceutical methods. For example, the composition can be used mainly parenterally, as an injection (intravenous injection, drip infusion, etc.), capsule, liquid, film-coated agent, or the like.

In formulating these, the composition can be appropriately combined with pharmacologically acceptable carriers or vehicles, such as sterile water, or physiological saline, phosphate-buffered saline (PBS), culture media (RPMI, AIM-V, X-VIVO10, etc.) and the like , vegetable oil, solvent, base, emulsifier, suspending agent, surfactant, stabilizer (e.g., human serum albumin), vehicle, preservative, binder, diluent, isotonicity agent, soothing agent, bulking agent, disintegrant, buffer, coating agent, lubricant, colorant, solubilizer, or other additives. It may also be used in combination with known pharmaceutical compositions or immunostimulants used in the treatment or prevention of the aforementioned diseases.

The pharmaceutical composition of the present invention may be cultured in an appropriate medium before administration to a subject. Stimulating molecules may also be added to the medium to maintain and amplify the activation and/or proliferation of T cells. Furthermore, serum or plasma may be added to the medium. The amount thereof to be added to the medium is not particularly limited, and may be, for example, 0% to 20% by volume. The amount of serum or plasma to be used can be changed according to the culture stage. For example, the serum or plasma concentration can be gradually reduced. The serum or plasma may be derived from either autologous or non-autologous source. While autologous sources are preferred since immune rejection is unlikely to occur, non-autologous sources are also useful because preparation is rapid and cell numbers can be ensured.

The pharmaceutical composition of the present invention is preferably administered parenterally to a subject. The method for parenteral administration includes intravenous, intraarterial, intramuscular, intraperitoneal, and subcutaneous administrations. The dosage is selected appropriately according to the condition, body weight, age, symptoms of the subject, the type of composition, and the like. The composition is generally administered to a subject weighing 60 kg such that the cell count is generally 1×10⁶ to 1×10¹⁰, preferably 1×10⁷ to 1×10⁹, more preferably 5×10⁷ to 5×10⁸, per administration. It may be administered in a single dose or in multiple doses.

### [Example]

The present invention is further described below with reference to Examples; however, the present invention is not limited to these Examples.

### Reference Example 1: Production of CD4 positive T cells (regulatory T cells) from HTLV-1 infected CD4 positive T cells

### (1) Isolation of HTLV-1 infected CD4+ T cell

Peripheral blood mononuclear cells were isolated from the peripheral blood of an ATL patient and subjected to positive selection with CD4 beads to isolate CD4+ T cells. The phenotype of the obtained T cells was analyzed by flow cytometry using various cell surface markers and confirmed to be strongly positive for CD3, CD4, and CD25.

### (2) Establishment of T-iPSCs

The HTLV-1 infected CD4+ T cells obtained in (1) were transfected with a Sendai virus (SeV) vector (SeVp [KOSM302L]) containing nuclear reprogramming factors (Oct4, Sox2, Klf4, c-Myc) and a SeV vector containing a nucleic acid encoding the SV40 large T antigen for gene transfer.

After gene transfer, the T cells were transferred to a 6-well plate coated with iMatrix and cultured in T cell medium (medium composition: RPMI, 10% human AB serum) supplemented with IL-2 in a CO₂ incubator.

The day after gene transfer, an equal volume of iPS medium (StemFit AK03N) was added, and thereafter half the volume was replaced with StemFit AK03N every other day.

T-iPSC colonies could be observed 21 days later, after which colony pickup was performed.

### (3) Induction of differentiation from CD4T-iPSCs to CD4 positive cells

The T-iPSC colonies obtained in (2) were finely disrupted and cultured on 10T1/2 feeder cells in a medium containing VEGF, FBS, insulin, transferrin, L-glutamine, α-monothioglycerol, ascorbic acid, etc., for the first week in hypoxic culture and for the second week in an incubator with a 20% oxygen concentration. They were then cultured for additional four weeks on 10T1/2 feeder cells expressing Notch Ligand, together with cytokines (IL-7, FLT3L, SCF), while replacing the feeder cells with fresh ones once a week. After four weeks, all non-adherent cells were collected and T cell receptor (TCR) stimulation was performed. TCR stimulation was performed about every two weeks with X-ray irradiated allogeneic peripheral blood mononuclear cells and PHA, or anti-CD3/CD28 antibodies.

The phenotype of T cells obtained by differentiation induction from CD4T-iPSCs was analyzed by intracellular staining and flow cytometry. As a result, the CD4+ T cells induced from HTLV-1 infected CD4T-iPSs were FOXP3 positive and CD25 positive, and showed a regulatory T cell-like phenotype.

### Example 1

The gene expression of the ATL-iPSC-derived CD4+ T cells obtained in the above Reference Example was compared with that of CD4T-iPSC-derived CD8+ T cells established from CD4T cells of a healthy subject by single-cell RNA sequencing analysis.

For single-cell RNA sequencing analysis, emulsions were prepared and RNA was extracted from each of ATL-iPSC-derived CD4+ T cells and CD4T-iPSC-derived CD8+ T cells, using the Chromium Next Gem Single Cell 3' Reagent Kits v3.1 (Dual Index) and the 10x Chromium Controller System, according to the 10x Genomics protocol, and barcodes were further assigned. Libraries were then prepared, and samples were evaluated using Bioanalyzer 2100. Thereafter, sequencing was outsourced to an external laboratory using HiseqX (Illumina, San Diego, CA). Fastq files were aligned to GRCh38 by using Cell Ranger (v7.0.0) (10x Genomics). RNA data normalization and downstream analysis were performed using the Seurat R package (version 4.3.0), which enables integrated processing of multimodal single-cell dataset.

Fig. 1 shows the comparison results of the expression of RUNX3, RUNX1, ZBTB7B, SOCS1, SOCS3, and GIMAP5 between CD4T-iPSC-derived CD8+ T cells and ATL-iPSC-derived CD4+ T cells using UMAP. In ATL-iPSC-derived CD4+ T cells, RUNX3, ZBTB7B, SOCS1, SOCS3, and GIMAP5 showed low expression, while RUNX1 showed high expression. On the other hand, in CD4T-iPSC-derived CD8+ T cells, these five genes were highly expressed and RUNX1 was lowly expressed.

### Example 2

The genes identified by the differential expression analysis in Example 1 suggest a possible involvement in CD4/CD8 lineage selection. The present inventors hypothesized that knocking out or knocking in these genes in T-iPSCs might enable differentiation of T-iPSCs into CD4+ T cells. Therefore, using the aforementioned genome editing method, one of the identified genes, RUNX3, was knocked out by nucleofecting gRNA with CRISPR/Cas9.

After knockout, T-iPSCs were cultured for 7-10 days and then reseeded to form single cells. Colonies derived from single cells were picked and continuously cultured, and genomic DNA was extracted. Genotyping PCR was performed using the extracted genomic DNA as a template, and the obtained PCR products were Sanger sequenced. The information obtained by Sanger sequencing confirmed that the deletion of one base or the insertion of two bases, or the deletion of two bases or the insertion of one base, results in an amino acid mutation downstream of the confirmed insertion or deletion site, and that due to the occurrence of a stop codon midway, the gene is shorter than the wild-type gene, and the amino acid mutation causes incomplete translation.

The obtained RUNX3 knockout T-iPSCs were induced to differentiate into T cells by a method similar to Reference Example 1 (3). After the start of differentiation induction, the expression of CD4 and CD8 was monitored over time by flow cytometry. The results are shown in Fig. 2. Three weeks after the start of differentiation induction, CD4-/CD8- cell population and CD4+/CD8- cell population were present, and the CD4+/CD8- cell population subsequently increased. Flow cytometry at 10 weeks shows the state after CD4 selection using CD4 beads, and CD4+/CD8- iPSC-derived CD4+ T cells were successfully obtained.

For comparison, Fig. 3 shows time-course changes of the CD4/CD8 ratio by flow cytometry during differentiation induction of hematopoietic progenitor cells derived from wild-type CD4T-iPSCs. After extraction, hematopoietic progenitor cells were continuously cultured on feeder cells, and flow cytometry was performed every week using T cell surface antigen markers. Three weeks after the start of differentiation induction, the CD4-/CD8- cell population was predominant, and a CD8+ cell population predominantly emerged from the eighth week onwards.

### Example 3

To demonstrate that the expression level of RUNX3 determines the induction of differentiation to CD4/CD8, RUNX3 was overexpressed in ATL-iPSCs, which originally had low RUNX3 expression and could generate CD4+ T cells upon differentiation induction, and it was examined which of CD4 and CD8 cells were obtained by differentiation induction. ATL-iPSCs were induced to differentiate, and at the stage of hematopoietic progenitor cell, RUNX3 was overexpressed using a retroviral vector. Differentiation was then performed in the same manner as before, and the CD4/CD8 ratio was analyzed over time by flow cytometry.

The results are shown in Fig. 4. Three weeks after the start of differentiation induction, the CD4-/CD8- cell population was predominant, but from the ninth week onwards, a CD8+ cell population predominantly emerged. In other words, without RUNX3 overexpression, ATL-iPSCs differentiated into CD4+ T cells, but with RUNX3 overexpression, they were induced to differentiate into CD8+ T cells. This demonstrates that high levels of RUNX3 expression induce differentiation into CD8+ T cells, while low levels of RUNX3 expression induce differentiation into CD4+ T cells.

### Example 4

CD4+ T cells obtained by differentiation induction from T-iPSCs after RUNX3 knockout were analyzed by flow cytometry using antibodies against Foxp3, which is characteristic of regulatory T cell, and CD45RA, which is a naive cell marker.

Fig. 5 shows the results of flow cytometry analysis of CD4+ T cells derived from RUNX3 KO CD4T-iPSCs, using anti-CD45RA and anti-Foxp3 antibodies. The CD4+ T cells derived from RUNX3 KO CD4T-iPSCs were CD4+, CD45RA-, and Foxp3-. On the other hand, the CD4+ T cells derived from ATL-iPSCs showed a CD4+, CD45RA-, and Foxp3+ fraction, and a CD4+, CD45RA+, and Foxp3low fraction. That is, RUNX3-knockout T-iPSC-derived CD4+ T cells did not exhibit the fractions of naive regulatory T cells (CD45RA+, Foxp3low) or activated regulatory T cells (CD45RA-, Foxp3high) seen in ATL-iPSC-CD4+ T cells. From the above, it could be confirmed that RUNX3-knockout T-iPSC-derived CD4+ T cells are not regulatory T cells.

### Example 5

LMP2-specific CTLs were isolated by a conventional method from peripheral blood mononuclear cells derived from healthy individual. By the same method as in Reference Example 1, iPSCs were induced from LMP2-specific CTLs and RUNX3 was knocked out in the same manner as in Example 2. Wild-type iPSCs derived from LMP2-specific CTLs and RUNX3-knockout iPSCs derived from LMP2-specific CTLs were each induced to differentiate into T cells by a method similar to that in Reference Example 1 (3), and analyzed by flow cytometry using anti-CD4 antibody or anti-CD8 antibody, and LMP2-class I tetramer.

The results are shown in Fig. 6. When T cells are induced to differentiate from iPSCs derived from antigen-specific LMP2-CTLs, they generally differentiate into CD8SP cells (Fig. 6, left). However, by knocking out RUNX3, they differentiated into CD4SP cells (Fig. 6, right). While the original LMP2-specific CTLs were class I-restricted CD8SP CTLs, by knocking out RUNX3, they differentiated into CD4SP while maintaining class I-restricted LMP2 antigen specificity.

### Example 6

The antigen-specific cytotoxic activity of RUNX3 KO LMP2-iPSC-T cells prepared in Example 5 was evaluated by chromium assay using three types of LMP2-expressing cells (lymphoma-derived NK-YS, ENKL-J1, and EBV-infected T lymphocyte, CAEBV). As a positive control, LMP2-iPSC-T cells (CD8+) were used, and primary ATL cells were used as a negative control.

The results are shown in Fig. 7. RUNX3 KO LMP2-iPSC-T cells had antigen-specific cytotoxic activity, though it was inferior to that of LMP2-iPSC-T cells.

The IFNγ secretion ability of RUNX3 KO LMP2-iPSC-T cells was therefore evaluated by ELISPOT assay. Furthermore, the production of the cytotoxic cytokines perforin and granzyme B was evaluated by flow cytometry.

The results are shown in Fig. 8. RUNX3 KO LMP2-iPSC-T cells highly secreted IFNγ in response to antigen stimulation, at levels comparable to LMP2-iPSC-T cells (Fig. 8A). They were also shown to produce perforin and granzyme B in response to antigen stimulation (Fig. 8B).

### Example 7

The ability of RUNX3 KO LMP2-iPSC-T cells to secrete various cytokines was examined. The results are shown in Fig. 9. In response to antigen stimulation, RUNX3 KO LMP2-iPSC-T cells highly secreted IL-2, which is secreted by Th1 cells, and also highly secreted IL-4 and IL-10, in addition to cytokines highly secreted by CTLs, such as IFNγ and TNF-α.

### Example 8

In order to further evaluate whether RUNX3 KO LMP2-iPSC-T cells exhibit Th1 cell phenotypes, the expression of transcription factors and cell surface molecules characteristic of Th1 cells was examined.

The results are shown in Fig. 10. RUNX3 KO LMP2-iPSC-T cells highly expressed TBX21, STAT1, and STAT4 (Fig. 10A), and were positive for CD107a and CD366 (Fig. 10B). From the above results, it was clarified that RUNX3 KO LMP2-iPSC-T cells exhibit Th1 cell phenotypes.

### Example 9

HPV16E6-specific CTLs were isolated from peripheral blood mononuclear cells of healthy individuals by a conventional method. By the same method as in Reference Example 1, iPSCs were induced from HPV16E6-specific CTLs, and RUNX3 was knocked out by the same method as in Example 2. RUNX3 KO HPV16E6-iPSCs were induced to differentiate into T cells by the same method as in Reference Example 1 (3). The cytotoxic activity of the obtained RUNX3 KO HPV16E6-iPSC-T cells used in combination with HPV16E6-iPSC-T cells (CD8+) induced to differentiate from wild-type HPV16E6-iPSC was evaluated by chromium assay in the same manner as in Example 6.

The results are shown in Fig. 11. HPV16E6-iPSC-T cells exhibited cytotoxic activity specific to HPV16E6-expressing cervical cancer cell line SiHa (Series 2), and this cytotoxic activity was further enhanced by co-culture with RUNX3 KO HPV16E6-iPSC-T cells (Series 1).

### Example 10

GD2-targeting chimeric antigen receptor-T cells (GD2-CARTs) were generated from peripheral blood mononuclear cells derived from healthy individuals by a conventional method (Kinoshita et al., Cancer Research Communications 2024). The cytotoxic activity of the RUNX3 KO LMP2-iPSC-T cells generated in Example 5 in combination with the GD2-CAR-T cells obtained above was evaluated by chromium assay in the same manner as in Example 6. Cell proliferation was also compared between culture of each cell alone and co-culture of the two.

The results are shown in Fig. 12. Compared to when GD2-specific CAR-T cells (GD2-CARTs) and RUNX3 KO LMP2-iPSC-T cells (CD4rejTs) were each cultured at 1x10⁶ for 72 hr, the cell number significantly increased in co-cultured conditions (1:1=0.5x10⁶:0.5x10⁶) (Fig. 12A).

Furthermore, the cytotoxic activity in GD2-CARTs cultured for 72 hr and in CD4rejTs and GD2-CARTs (1:1) co-cultured for 72 hr, against GD2-positive EBV-associated lymphoma cells was evaluated by chromium assay. As a result, the cytotoxic activity was enhanced more in the co-culture than CAR-T cells alone (Fig. 12B).

### Example 11

The combined effect of GD2-specific CAR-T cells and RUNX3 KO LMP2-iPSC-T cells was confirmed through animal experiments.

GD2-positive EBV-associated lymphoma cells were intraperitoneally transplanted into NOG mice each at 1x10⁶ cells. Five days later, effector cells obtained by 72 hr monoculture of GD2-CARTs or 72 hr coculture of GD2-CARTs + CD4rejTs (1:1) were intraperitoneally injected at 4x10⁶ cells, and tumor growth was monitored over time.

The results are shown in Fig. 13. After the elapse of more than three weeks from the administration of effector cells, tumor growth suppressive effect was more remarkable in mice with administration of GD2-CARTs + CD4rejTs than in mice with administration of GD2-CARTs alone.

### [Industrial Applicability]

Since the iPSC-derived CD4+ T cells obtained by the present invention themselves retain cytotoxic activity and also function as Th1 cells, they are highly useful as immunotherapeutic agents for the treatment of cancer and infectious diseases, either alone or in combination with CAT-T, TCR-T cells, or CTLs.

This application is based on a patent application No. 2023-089785 filed in Japan (filing date: May 31, 2023), the contents of which are incorporated in full herein.

## Claims

1. A method for producing an induced pluripotent stem cell (iPSC)-derived CD4+ T cell, comprising a step of knocking out one or more genes selected from the group consisting of RUNX3, ZBTB7B, SOCS1, SOCS3, and GIMAP5 and/or overexpressing the RUNX1 gene in a human iPSC.

2. The method according to claim 1, wherein the iPSC is derived from an antigen-specific T cell, a peripheral blood-derived T cell, a peripheral blood mononuclear cell, or a fibroblast.

3. The method according to claim 1 or 2, wherein the gene to be knocked out includes RUNX3.

4. The method according to any one of claims 1 to 3, wherein the CD4+ T cell is CD4 single-positive.

5. The method according to any one of claims 2 to 4, wherein the antigen-specific T cell is specific for an antigen peptide presented on a cancer cell or a virus-infected cell, or the peripheral blood-derived T cell or peripheral blood mononuclear cell is derived from a cancer patient or a virus-infected individual.

6. The method according to any one of claims 2 to 5, wherein the antigen-specific T cell or peripheral blood-derived T cell is a CD8+ T cell.

7. The method according to any one of claims 2 to 5, wherein the antigen-specific T cell or peripheral blood-derived T cell is a CD4+ T cell.

8. A human CD4+ T cell obtained by the method according to any one of claims 1 to 7.

9. A human iPSC-derived T cell having the following properties:
(a) being CD4 single-positive;
(b) having a TCR specific to an antigen peptide presented on a cancer cell or a virus-infected cell;
(c) exhibiting one or more of the following Th1 cell phenotypes:
(c1) highly secreting IFNγ and IL-2;
(c2) highly expressing one or more genes selected from the group consisting of TBX21, STAT1, and STAT4;
(c3) expressing CD107a and CD366;
(d) independently having cytotoxic activity specific to the antigen peptide of (b); and
(e) enhancing the proliferation and/or cytotoxic activity of cancer antigen- or viral antigen-specific CTL or CAR-T cell.

10. The human iPSC-derived T cell according to claim 9, in which one or more genes selected from the group consisting of RUNX3, ZBTB7B, SOCS1, SOCS3, and GIMAP5 are knocked out and/or the RUNX1 gene is overexpressed.

11. A pharmaceutical composition comprising the human iPSC-derived CD4+ T cell according to any one of claims 8 to 10.

12. The pharmaceutical composition according to claim 11, which is an immunotherapeutic agent.

13. The pharmaceutical composition according to claim 12, which is for treating cancer or a viral infectious disease.

14. The pharmaceutical composition according to claim 13, which is combined with cancer antigen- or viral antigen-specific CTL or CAR-T cell.

15. The pharmaceutical composition according to claim 13, which is combined with an antigen-presenting cell and/or an antigen peptide presented on a cancer cell or a virus-infected cell.
